# DEMANDE DE BREVET EUROPEEN

(11) **EP 3 141 470 A1**
(43) Date de publication de la demande: **15.03.2017**
(21) Numéro de dépôt: 16185782.6
(22) Date de dépôt: 25.08.2016
(51) Int. Cl.: B63B 21/00, B63B 21/08, B63B 21/50, E02B 3/24

(54) **DISPOSITIF D'AMARRAGE D'UN NAVIRE À UN POSTE D'AMARRAGE D'UN PONTON ET PONTON CORRESPONDANT**

(30) Priorité: 08.09.2015 FR 1558330
(71) Demandeur: SAVOYE, 21000 Dijon (FR)
(72) Inventeur: JEANNIN, Rémy, 21220 FIXIN (FR)
(74) Mandataire: Vidon Brevets & Stratégie

(57) **Abrégé**

L'invention concerne un dispositif d'amarrage (1) d'un navire (2) à un poste d'amarrage (3) comprenant :
- des moyens d'engagement (20) portés par ledit navire (2) ou ledit poste d'amarrage (3), et
- des moyens de verrouillage (5) portés par ledit poste d'amarrage (3) ou ledit navire (2) respectivement, lesdits moyens de verrouillage (5) comprennent deux barres d'accroche (53a, 53b) espacées et s'étendant verticalement, au moins une partie des moyens d'engagement (20) étant aptes à venir s'engager entre les deux barres de façon à être maintenus par lesdits moyens de verrouillage (5) et à permettre l'amarrage du navire (2) au poste d'amarrage (3). Selon l'invention, lesdites barres d'accroche (53a, 53b) sont mobiles l'une par
rapport à l'autre entre une position de verrouillage dans laquelle les barres d'accroche (53a, 53b) sont rapprochées et maintiennent lesdits moyens d'engagement (20), et une position de déverrouillage dans laquelle les barres d'accroche (53a, 53b) sont écartées permettant le retrait ou l'insertion desdits moyens d'engagement (20).

## Description

### 1. DOMAINE DE L'INVENTION

L'invention concerne un dispositif d'amarrage d'un navire à un poste d'amarrage d'un ponton ou d'un quai, par exemple.

L'invention concerne, par ailleurs, un ponton équipé d'un ou plusieurs dispositifs d'amarrage.

La présente invention peut notamment être mise en oeuvre en mer, en lac ou en rivière, et est particulièrement, mais non exclusivement, adaptée aux bateaux de plaisance.

### 2. ARRIÈRE-PLAN TECHNOLOGIQUE

L'amarrage d'un navire, par exemple un bateau de plaisance ou un voilier, est la dernière phase d'une manoeuvre d'accostage. Elle consiste à maintenir le navire contre un ponton, par exemple, et à le relier à ce dernier afin de limiter les mouvements du navire par rapport au ponton.

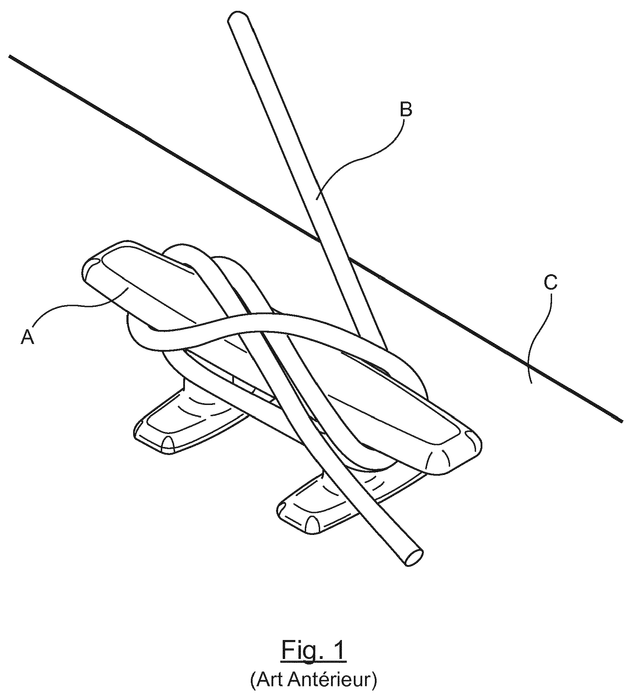

Une technique connue, illustrée sur la figure 1, consiste à relier le navire (non représenté) à un ponton C par la mise en oeuvre d'un cordage B et d'un ou plusieurs points d'amarrage A pouvant prendre la forme d'un taquet, une bitte ou un anneau. Le nombre de points d'amarrage est fonction de la taille du navire.

Un inconvénient de cette approche est qu'elle nécessite la présence d'une ou de plusieurs personnes. En outre, le navire étant soumis aux mouvements de l'eau (houle), une telle manoeuvre d'amarrage présente des risques pour les personnes présentes. Par ailleurs, un tel amarrage est peu sécurisé et il existe un risque relativement important de vol du navire.

Pour pallier certains de ces inconvénients, la demande de brevet WO2009/073897 propose la mise en oeuvre d'un mécanisme de verrouillage qui comprend une barre verticale solidaire d'un ponton, et disposée entre des éléments de guidage de l'étrave d'un bateau ayant une forme générale en V. Un mousqueton situé sur l'étrave du bateau est destiné à venir agripper la barre verticale de façon à amarrer de manière réversible le navire au ponton.

Par ailleurs, le ponton présente une articulation qui permet au mécanisme de verrouillage de suivre les mouvements de l'eau (dus à la marée et/ou à la houle).

Cette solution met en oeuvre de nombreuses pièces mécaniques qui rendent la fabrication complexe et coûteuse, et qui multiplient également les risques de dysfonctionnement.

Il existe donc un besoin important d'amélioration des dispositifs d'amarrage de navires, pour optimiser la sécurité, les coûts de fabrication et la maintenance, de façon simple et efficace.

### 3. OBJECTIFS DE L'INVENTION

L'invention, dans au moins un mode de réalisation, a notamment pour objectif de pallier au moins certains inconvénients de l'état de la technique.

Plus précisément, dans au moins un mode de réalisation de l'invention, un objectif est de faciliter l'amarrage d'un bateau à un poste d'amarrage d'un quai ou d'un ponton, notamment pour un utilisateur (un plaisancier notamment) qui serait seul à bord.

Un autre objectif d'au moins un mode de réalisation de l'invention est de fournir un dispositif d'amarrage qui soit de conception simple (et donc peu coûteux), fiable, robuste, sécurisé et qui supprime, ou à tout le moins, minimise les risques pour l'utilisateur.

Encore un autre objectif d'au moins un mode de réalisation de l'invention est de fournir un tel dispositif d'amarrage qui puisse s'adapter aux mouvements de l'eau et aux tailles variables des navires.

### 4. EXPOSÉ DE L'INVENTION

L'invention parvient à remplir tout ou partie de ces objectifs grâce à un dispositif d'amarrage d'un navire à un poste d'amarrage comprenant :
- des moyens d'engagement portés par le navire ou le poste d'amarrage, et
- des moyens de verrouillage portés par le poste d'amarrage ou le navire respectivement, lesdits moyens de verrouillage comprenant deux barres d'accroche espacées et s'étendant verticalement, au moins une partie des moyens d'engagement étant aptes à venir s'engager entre les deux barres de façon à être maintenus par lesdits moyens de verrouillage et à permettre l'amarrage du navire au poste d'amarrage.

Selon l'invention, lesdites barres d'accroche sont mobiles l'une par rapport à l'autre entre une position de verrouillage (fermée), dans laquelle les barres d'accroche sont rapprochées et maintiennent lesdits moyens d'engagement, et une position de déverrouillage (ouverte), dans laquelle les barres d'accroche sont écartées permettant le retrait (ou libération) ou l'insertion desdits moyens d'engagement.

Ainsi, la mise en oeuvre de barres d'accroche mobiles facilite l'amarrage et la libération d'un navire à un poste d'amarrage situé sur un ponton, par exemple.

Les barres d'accroche sont reliées entre elles par le biais d'au moins une liaison pivot permettant un mouvement des mâchoires l'une par rapport à l'autre.

Les moyens d'engagement, par exemple portés par le navire, peuvent être placés entre les barres d'accroche ouvertes, puis une fois ces dernières fermées, être maintenus par les barres d'accroche, permettant un amarrage aisé et fiable du navire au ponton. L'ouverture des barres d'accroche autorise le retrait des moyens d'engagement et l'éloignement du navire par rapport au ponton. Les barres d'accroche, qui sont par exemple solidaires du ponton, s'étendent dans un plan perpendiculaire à la surface du ponton et sont, par exemple, reliées entre elles au moins à une de leurs extrémités. De préférence, elles sont reliées entre elles à chacune de leurs extrémités de manière à assurer un amarrage fiable du navire.

Dans une alternative, les barres d'accroche sont fixées sur le navire alors que les moyens d'engagement sont solidaires du ponton.

La mise en oeuvre de telles barres d'accroche offre un degré de liberté aux moyens d'engagement disposés et retenus entre les barres d'accroche et permet de diminuer les forces appliquées sur le dispositif d'amarrage en cas de mer agitée, par exemple. De par leur forme, les barres d'accroche permettent en effet aux moyens d'engagement de se déplacer verticalement entre ces dernières de manière à suivre les mouvements de l'eau (dus à la marée et/ou à la houle).

L'espace entre les barres d'accroche permet en outre un jeu latéral des moyens d'engagement.

Par ailleurs, ces barres d'accroche sont adaptées pour recevoir une variété de hauteur de moyens d'engagement, cette hauteur étant fonction du type de bateau.

Un tel dispositif d'amarrage présente une structure et un fonctionnement qui sont simples, et facilite la manoeuvre d'accostage et d'amarrage d'un navire à un ponton (et inversement, le désamarrage du navire).

L'amarrage du navire est en outre sécurisé.

Par ailleurs, un tel dispositif d'amarrage peut être aisément adapté sur un ponton existant et sur tout type de navire.

Selon un aspect particulier de l'invention, lesdits moyens d'engagement comprennent une première portion d'extrémité reliée par une portion centrale de largeur/section réduite à une deuxième portion d'extrémité, cette dernière étant reliée au navire ou au poste d'amarrage.

Ainsi, les moyens d'engagement comprennent une première portion d'extrémité plus large que la portion centrale. Par exemple, il peut s'agir d'un élément en forme de « T », la barre horizontale du « T » étant la première portion d'extrémité et la barre verticale du « T », la portion centrale de largeur réduite.

D'autres formes peuvent être mises en oeuvre, comme par exemple une forme en « Y ».

Selon un aspect particulier de l'invention, ladite deuxième portion d'extrémité présente une lumière destinée à coopérer avec une perche d'amarrage ou d'éloignement du navire.

Ainsi, la deuxième portion d'extrémité des moyens d'engagement, reliée au navire par exemple, présente une lumière destinée à coopérer avec une perche maniée par un plaisancier situé sur le ponton de manière à placer les moyens d'engagement entre les barres d'accroche lors de l'amarrage, ou à repousser (ou éloigner) les moyens d'engagement à l'écart des barres d'accroche lors du démarrage du navire. Une telle perche peut être automatique.

Selon un aspect particulier de l'invention, dans la position de verrouillage des barres d'accroche, ces dernières sont situées de part et d'autre de ladite portion centrale.

Ainsi, dans cette position, les barres d'accroche viennent se refermer sur la portion centrale des moyens d'engagement, ce qui permet de sécuriser l'amarrage du navire.

Selon un aspect particulier de l'invention, ladite première portion d'extrémité est disposée sur un premier côté des barres d'accroche, l'écart entre les barres d'accroche dans la position de verrouillage étant inférieur à la largeur de ladite première portion d'extrémité.

Selon un aspect particulier de l'invention, l'écart entre les barres d'accroche dans la position de verrouillage est supérieur à la largeur de ladite portion centrale.

Selon un aspect particulier de l'invention, l'écart entre les barres d'accroche dans la position de verrouillage est inférieur à la largeur de ladite deuxième portion d'extrémité.

Ainsi, dans la position verrouillée des barres d'accroche, l'écart entre ces dernières est inférieur à la largeur des première et deuxième portions d'extrémités des moyens d'engagement. Par conséquent, les barres d'accroche se retrouvent bloquées entre la première et la deuxième portion d'extrémité des moyens d'engagement. Une telle configuration permet de relier solidement le navire au poste d'amarrage et de supprimer, ou à tout le moins limiter, les mouvements du navire selon un axe perpendiculaire à l'axe des barres d'accroche.

Par ailleurs, dans cette position verrouillée, l'écart entre les barres d'accroche est supérieur à la largeur de la portion centrale et permet donc un déplacement vertical, c'est-à-dire selon un axe parallèle à l'axe des barres d'accroche, des moyens d'engagement entre les barres d'accroche. Cette configuration permet donc au navire de se déplacer verticalement selon les mouvements de l'eau (houle, marée) et ainsi de limiter les contraintes/forces mécaniques appliquées sur le dispositif.

Selon un aspect particulier de l'invention, les moyens d'engagement comprennent une première partie fixe solidaire du navire ou du poste d'amarrage, et une deuxième partie montée de façon amovible sur la première partie fixe et destinée à coopérer avec les moyens de verrouillage.

La mise en oeuvre d'une telle partie amovible permet d'insérer manuellement, la première portion d'extrémité des moyens d'engagement entre les barres d'accroche des moyens de verrouillage sans avoir à déplacer les barres entre la position ouverte (déverrouillée) et la position fermée (verrouillée). De tels moyens d'engagement sont compatibles avec un dispositif qui présenterait des barres d'accroche mobiles (dans ce cas, il n'est pas nécessaire de désolidariser la partie amovible) mais aussi avec un dispositif dont les barres d'accroche seraient fixes.

Selon un autre aspect particulier de l'invention, les moyens d'engagement comprennent une première partie fixe solidaire du navire ou du poste d'amarrage, et une deuxième partie montée de façon pivotante sur la première partie fixe et destinée à coopérer avec les moyens de verrouillage.

La mise en oeuvre d'une telle partie pivotante permet d'insérer la première portion d'extrémité des moyens d'engagement entre les barres des moyens de verrouillage sans avoir à déplacer les barres entre la position ouverte/déverrouillée et la position fermée/verrouillée. Il est uniquement nécessaire de faire pivoter de 90° (position verticale) cette partie pivotante afin de faire passer la première partie d'extrémité des moyens d'engagement au-delà des barres d'accroche, puis de pivoter la partie pivotante de nouveau de 90° (position horizontale) pour bloquer les moyens d'engagement entre les barres d'accroche et ainsi permettre l'amarrage sécurisé du navire.

De tels moyens d'engagement sont également compatibles avec un dispositif qui présenterait des barres d'accroche mobiles ou des barres d'accroche fixes.

Selon un aspect particulier de l'invention, chaque barre d'accroche porte à au moins une de ses extrémités une équerre montée pivotante avec une équerre correspondante de l'autre barre d'accroche, les barres d'accroche et les équerres formant ainsi deux mâchoires mobiles.

La mise en oeuvre d'équerres à une ou à chacune des extrémités des barres d'accroche permet de former deux mâchoires qui permettent d'assurer un amarrage sécurisé d'un navire à un poste d'amarrage, de façon simple et peu coûteuse.

Selon un aspect particulier de l'invention, lesdits moyens de verrouillage comprennent des moyens d'actionnement agissant sur au moins une équerre de chaque mâchoire.

Selon un aspect particulier de l'invention, lesdits moyens d'actionnement comprennent une paire de bielles montées pivotantes autour d'un même axe à une première de leurs extrémités, chacune desdites bielles étant montée pivotante, à une deuxième extrémité, sur une équerre distincte d'une desdites mâchoires.

Selon un aspect particulier de l'invention, lesdites bielles sont montées pivotantes à leur première extrémité sur des moyens de support mobiles en translation de manière à faire varier l'angle d'ouverture entre les deux bielles.

Selon un aspect particulier de l'invention, les moyens de support sont reliés à une came d'actionnement par une tige.

Selon un aspect particulier de l'invention, ladite came est déplacée par un moteur.

Ainsi, l'ouverture et la fermeture des mâchoires sont automatiques et permettent de faciliter la manoeuvre d'amarrage.

Dans une variante de l'invention, ladite came est déplacée par une poignée reliée à ladite came par un axe.

Ainsi, l'ouverture et la fermeture des mâchoires peuvent se faire de manière manuelle.

Selon un aspect particulier de l'invention, le dispositif comprend en outre des moyens de centrage et de guidage des moyens d'engagement vers les moyens de verrouillage, situés de part et d'autres des barres d'accroche.

Ainsi, de tels moyens de centrage et de guidage permettent de simplifier l'engagement des moyens d'engagement dans les moyens de verrouillage de manière à faciliter la manoeuvre d'amarrage. Ces moyens de guidage sont utiles pour un plaisancier qui serait seul à bord ou lors de conditions météorologiques difficiles, notamment.

Selon un aspect particulier de l'invention, lesdits moyens de verrouillage sont mobiles par rapport audit poste d'amarrage lorsqu'ils sont portés par ce dernier.

Selon un aspect particulier de l'invention, lesdits moyens de verrouillage sont portés par au moins un chariot apte à se déplacer en translation sur un rail, ledit rail étant monté fixe sur le poste d'amarrage.

Ainsi, le dispositif d'amarrage comprend des moyens de déplacement des moyens de verrouillage sur le poste d'amarrage, un ponton par exemple. De tels moyens permettent une flexibilité accrue du dispositif.

Le dispositif d'amarrage de l'invention peut s'adapter à tout type d'installation destinée à accueillir des navires en mer, rivière, ou lac, notamment.

L'invention concerne également un ponton comprenant un ou plusieurs dispositifs d'amarrage tels que décrits précédemment.

### 5. LISTE DES FIGURES

D'autres caractéristiques et avantages de l'invention apparaîtront à la lecture de la description suivante, donnée à titre d'exemple indicatif et non limitatif, et des dessins annexés, dans lesquels :
- la figure 1 est une vue en perspective d'un dispositif d'amarrage tel que décrit dans l'art antérieur ;
- la figure 2 est une vue en perspective, d'un dispositif d'amarrage selon un premier mode de réalisation de l'invention, dans sa position inactive ;
- la figure 3 est une vue en perspective, du dispositif d'amarrage de la figure 2 dans sa position active ;
- la figure 4 est une vue d'ensemble, en perspective, d'un dispositif d'amarrage, selon un deuxième mode de réalisation de l'invention ;
- la figure 5 est une vue de détail, et en perspective, du dispositif d'amarrage de la figure 4, dans sa position active ;
- la figure 6 est une vue en perspective du dispositif d'amarrage des figures 4 et 5, et
- la figure 7 illustre une variante des moyens d'actionnement d'un dispositif d'amarrage conforme à l'invention ;
- les figures 8A et 8B sont des vues en perspective, d'un dispositif d'amarrage selon un troisième mode de réalisation de l'invention ;
- les figures 9A et 9B sont des vues en perspective, d'un dispositif d'amarrage selon un quatrième mode de réalisation de l'invention.

### 6. DESCRIPTION DÉTAILLÉE DE MODES DE REALISATION DE L'INVENTION

Le dispositif d'amarrage de l'invention comprend, dans les modes de réalisation décrits ci-après, des moyens de verrouillage, sous la forme de deux barres d'accroche espacées qui sont solidaires d'une installation portuaire (un ponton, par exemple), et des moyens d'engagement qui sont solidaires d'un navire (un bateau de plaisance, par exemple) à amarrer à l'installation portuaire et qui sont destinés à venir s'engager entre les barres d'accroche.

De par leur forme, les barres permettent en effet aux moyens d'engagement de se déplacer verticalement entre ces dernières de manière à suivre les mouvements de l'eau (dus à la marée et/ou à la houle).

Les premier et deuxième modes de réalisation décrits ci-après présentent un principe de fonctionnement identique dans lequel les moyens de verrouillage portés par le ponton comprennent deux mâchoires mobiles l'une par rapport à l'autre afin d'agripper les moyens d'engagement portés par un navire.

Les troisième et quatrième modes de réalisation décrivent un principe de fonctionnement différent des deux premiers modes de réalisation puisque les deux mâchoires sont fixes et ce sont les moyens d'engagement portés par un navire qui doivent être déplacés et/ou manipuler afin de coopérer avec les mâchoires du ponton.

### 6.1 Description d'un premier mode de réalisation

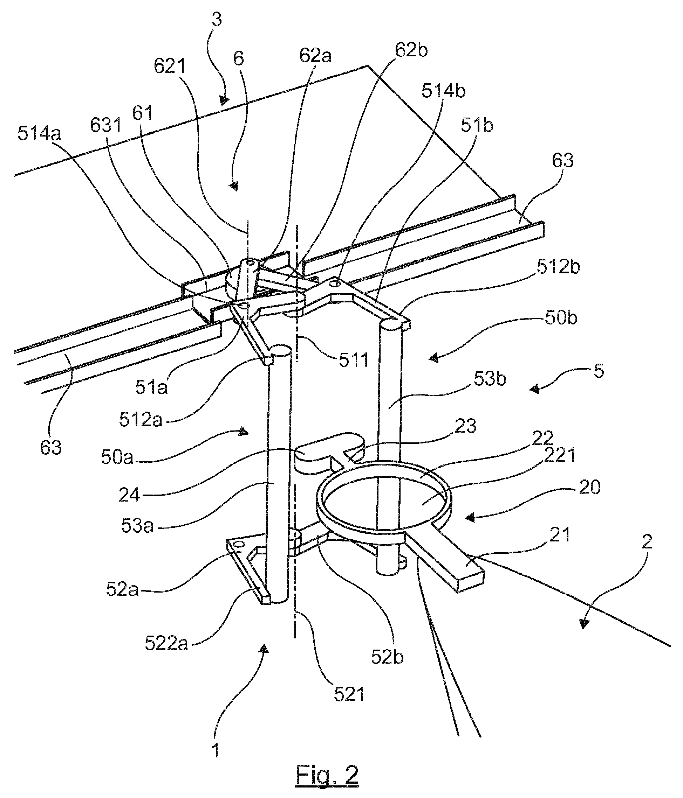

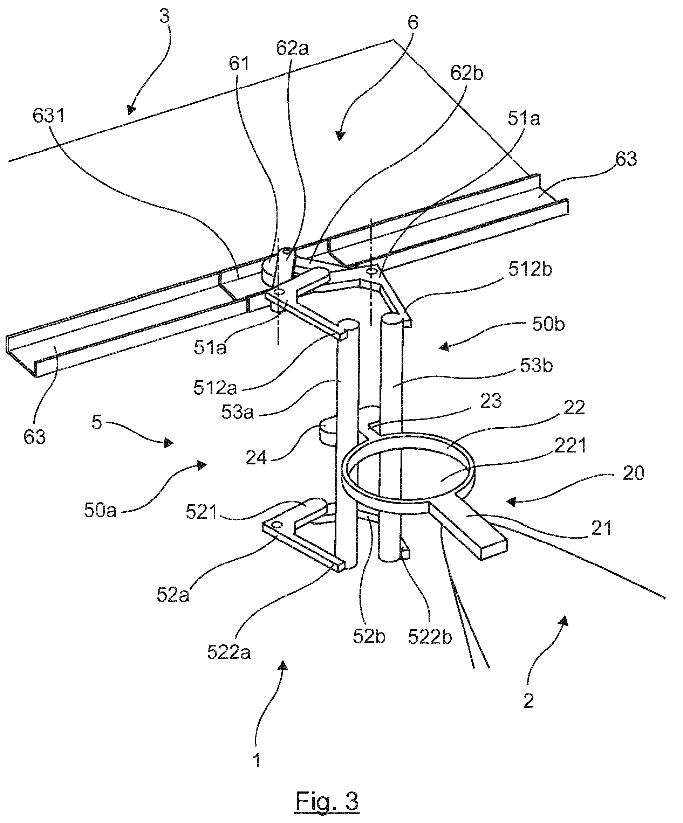

Un dispositif d'amarrage 1 d'un navire 2, selon un premier mode de réalisation, est partiellement représenté sur les vues en perspective des figures 2 et 3. Ce dispositif d'amarrage 1 est mis en oeuvre dans un port de plaisance, par exemple.

Il comprend des moyens de verrouillage 5, comprenant deux mâchoires d'accroche 50a et 50b, solidaires d'un ponton flottant 3, et des moyens d'engagement 20 solidaires d'un navire 2, qui est amarré au ponton 3 sur la figure 3.

Dans ce premier mode de réalisation, les moyens de verrouillage 5 sont solidarisés au ponton 3 par le biais d'un profilé 63, s'étendant longitudinalement le long du ponton 3. Le profilé 63 peut recevoir une pluralité de moyens de verrouillage, de manière à accueillir une pluralité de navires.

Le profilé 63, qui est solidaire de la surface supérieure du ponton 3, présente ici une section sensiblement en U. Bien évidemment, une section de forme différente pourrait être mise en oeuvre.

Le dispositif d'amarrage 1 comprend en outre des moyens d'actionnement 6 des moyens de verrouillage 5 comprenant un actionneur (non représenté), qui peut être commandé de façon manuelle ou automatique. L'actionneur agit sur une portion mobile 631 du profilé 63 permettant ainsi le déplacement de la portion mobile 631 du profilé 63 perpendiculairement à l'axe longitudinal du profilé 63, dans un même plan.

La portion mobile 631 porte un élément de support 61 fixe sur lequel sont montés articulés autour d'un même axe de rotation 621 deux leviers, ou bielles, 62a et 62b, qui s'étendent, en formant un V d'angle variable, en direction du navire 2 à amarrer.

Les bielles 62a et 62b sont respectivement solidaires à une extrémité d'une équerre supérieure 51a et 51b. Ces équerres 51a, 51b sont montées mobiles en rotation sur les leviers 62a et 62b respectivement autour d'un axe 513a, 513b situé au voisinage de leur coin/angle droit.

Par ailleurs, les équerres supérieures 51a et 51b sont reliées entres elles par l'intermédiaire d'une liaison pivot, ou axe de rotation, 511 situé à une première de leurs extrémités.

Les moyens de verrouillage 5 comprennent, en outre, une paire d'équerres inférieures 52a et 52b qui sont reliées entres par le biais d'une liaison pivot, ou axe de rotation, 521 situé à une première de leurs extrémités.

Les deux paires d'équerres 51a, 51b, 52a, 52b sont reliées entres elles par une paire de barres d'accroche 53a, 53b s'étendant dans un plan perpendiculaire au plan des équerres, des leviers 62a et 62b, et du profilé 63 (et du ponton 3).

Plus précisément, la première barre d'accroche 53a est fixée entre les extrémités respectives 512a, 522a des équerres 51a, 52a. De la même façon, la deuxième barre d'accroche 53b est fixée entre les extrémités respectives 512b et 522b (non visible) des équerres 51b et 52b.

Dans ce mode de réalisation, les équerres inférieures 52a, 52b ne sont pas solidarisées au ponton 3. Dans une variante, ces équerres 52a, 52b sont solidarisées au ponton d'une manière similaire aux équerres supérieures 51a et 51b.

L'ensemble constitué des barres 53a, 53b et des équerres 51a, 51b, 52a, 52b, forme ainsi deux mâchoires 50a, 50b mobiles entre une position ouverte (figure 2) et une position fermée (figure 3).

Ces mâchoires 50a, 50b peuvent se déplacer entre une position ouverte (comme illustrée sur la figure 2) dans laquelle les barres d'accroches 53a, 53b sont éloignées l'une de l'autre autorisant l'insertion ou le retrait des moyens d'engagement 20 (chaque paire d'équerres formant sensiblement un « W »), et une position fermée (comme illustrée sur la figure 3), dans laquelle les barres d'accroche 53a, 53b sont rapprochées et maintiennent les moyens d'engagement 20 portés par le navire 2 (chaque paire d'équerres formant sensiblement un « U »).

Les moyens de verrouillage 5 comprenant les mâchoires 50a, 50b sont donc destinés à coopérer avec les moyens d'engagement 20 solidaires de la pointe avant du navire 2.

Ces moyens d'engagement 20 comprennent un axe 21 qui est fixé au navire (par vissage, par exemple) et qui est prolongé par un élément 22 en forme d'anneau, présentant une lumière 221 intérieure. Un plaisancier situé sur le ponton peut ainsi introduire une gaffe ou une perche dans l'anneau 22 de façon à tirer et guider le navire 2 vers les moyens de verrouillage 5, ou à repousser le navire 2 à l'écart du ponton 3.

Dans une variante, il est envisagé de mettre en oeuvre une perche automatique (non représentée) pour guider un navire qui est actionnée automatiquement à l'approche du navire, par exemple. Cette perche peut être également configurée pour effectuer le mouvement inverse, c'est-à-dire pour pousser le navire à l'écart du ponton 3.

L'anneau 22 est prolongé par une tige, ou portion centrale 23, de section réduite, elle-même prolongé par un plot ou première portion d'extrémité 24 (en forme de galet oblong). L'anneau 22 forme une deuxième portion d'extrémité des moyens d'engagement 20.

La tige 23 et le plot 24 sont situés de manière diamétralement opposée à l'axe 21. Le plot 24 s'étend perpendiculairement à la tige 23, ces deux éléments formant un T qui est configuré pour coopérer avec les mâchoires 50a, 50b lorsque ces dernières sont fermées (figure 3).

Plus précisément, comme illustré sur la figure 3, les mâchoires 50a, 50b viennent se loger entre le plot 24 et l'anneau 22, de chaque côté de la tige 23. De préférence, il existe un jeu entre les mâchoires 50a, 50b et la tige 23 de façon à permettre un débattement latéral et vertical de la tige 23 (dans un plan parallèle au ponton 3 et dans un plan perpendiculaire à ce dernier).

On décrit maintenant plus en détail le fonctionnement des moyens de verrouillage 5 et des moyens d'engagement 20, en relation avec les figures 2 et 3. On se place dans le cadre d'une manoeuvre d'amarrage du navire 2 au ponton 3 qui est équipé du dispositif d'amarrage 1.

Lorsqu'aucun navire n'est amarré au dispositif d'amarrage 1, les moyens de verrouillage 5 se situent en position ouverte (ou déverrouillée) comme illustré sur la figure 2. Dans cette position, la portion mobile 631 du profilé 63 est décalée vers le ponton 3 par rapport à l'axe longitudinal du profilé 63. Les mâchoires 50a, 50b sont ouvertes, c'est-à-dire qu'elles sont éloignées l'une de l'autre. Dans cette position, la distance entre les mâchoires 50a, 50b est supérieure à la largeur du plot 24.

On comprend ici que la largeur du plot 24 est la distance mesurée entre les deux extrémités du plot 24 selon un axe perpendiculaire à la tige 23 et à l'axe 21.

Lorsque le navire 2 est en approche des moyens de verrouillage 5, un plaisancier situé sur le ponton 3 peut introduire une gaffe dans l'anneau 22 et ainsi diriger les moyens d'engagement 20 entre les mâchoires 50a et 50b ouvertes.

L'anneau 22 peut également coopérer avec une perche actionnée de façon automatique, comme souligné précédemment.

Une fois que le plot 24 et la tige 23 sont engagés entre les mâchoires 50a, 50b ouvertes (figure 2), l'actionneur (activé manuellement ou de manière automatique) entraine le déplacement de la portion mobile 631 du profilé 63 jusqu'à ce que la portion mobile 631 soit alignée avec l'axe longitudinal du profilé 63. Ce déplacement de la portion mobile 631 provoque le rapprochement des leviers 62a, 62b, et donc un pivotement des équerres 51a, 51b, 52a, 52b, autour des axes de rotation 511, 521, ce qui provoque la fermeture des mâchoires 50a, 50b et leur positionnement entre le plot 24 et l'anneau 22, de chaque côté de la tige 23 (figure 3).

Les mâchoires 50a, 50b sont alors en position verrouillée et le navire 2 est solidement amarré au ponton 3.

Dans la position fermée ou verrouillée des mâchoires 50a, 50b, l'écart entre les mâchoires 50a et 50b est inférieur à la largeur du plot 24 et à la largeur (ou diamètre) de l'anneau 22.

Dans cette même position, l'écart entre les mâchoires 50a, 50b, est supérieur à la largeur de la tige 23 de façon à permettre le mouvement des moyens d'engagement 20 le long des mâchoires 50a, 50b et permettre au navire 2 de suivre les mouvements de l'eau (oscillations des vagues notamment) sans endommager la liaison entre le navire 2 et le ponton 3.

### 6.2 Description d'un deuxième mode de réalisation

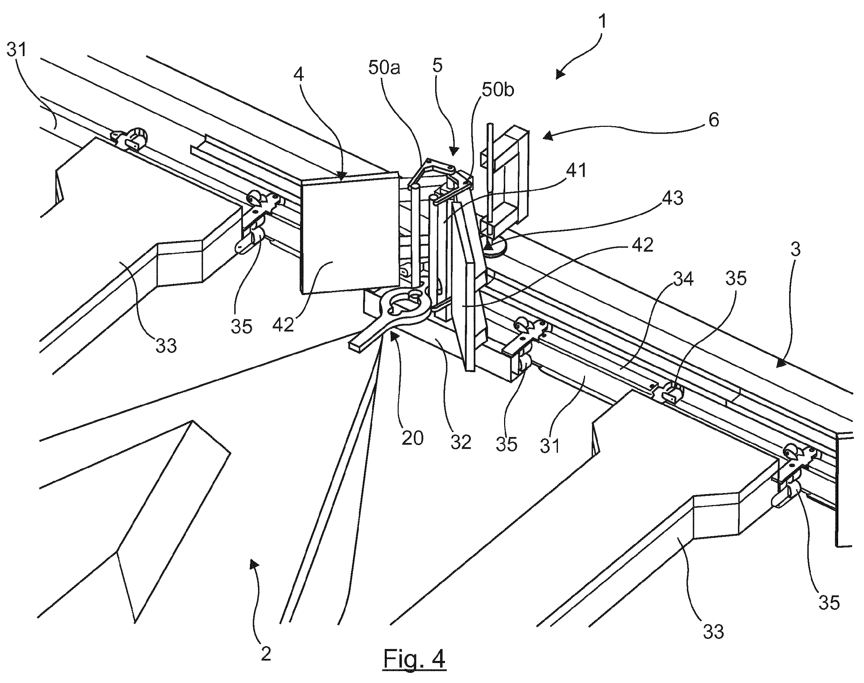

Un dispositif d'amarrage 1 d'un navire 2, selon un deuxième mode de réalisation, est représenté sur les figures 4 à 6.

Ce dispositif d'amarrage 1 comprend des moyens de verrouillage 5 comprenant deux mâchoires 50a et 50b (figure 4) solidaires d'un ponton flottant 3, et des moyens d'engagement 20 solidaires du navire 2 à amarrer au ponton 3.

Dans ce deuxième mode de réalisation, le ponton 3 comprend des moyens de déplacement des moyens de verrouillage 5 sur le ponton 3. Les moyens de déplacement comprennent un rail 31 qui s'étend sur au moins une partie de la longueur du ponton 3. Le rail 31 présente une section de forme sensiblement parallélépipédique et fait partie intégrante du ponton 3 (il peut être fixé de manière permanente à ce dernier dans une alternative).

Un chariot coulissant 32 est monté mobile en translation sur le rail 31 et est notamment destiné à supporter les moyens de verrouillage 5 du dispositif d'amarrage 1.

Le chariot 32 est relié en amont et en aval à un appontement flottant ("*catway*" en anglais) 33, s'étendant perpendiculairement au ponton 3, par l'intermédiaire d'une traverse 34, de telle sorte que l'ensemble du dispositif d'amarrage 1 puisse être déplacé le long du ponton 3 sur le rail 31.

Le déplacement de l'ensemble formé par le chariot 32 et les appontements 33 peut être effectué manuellement ou à l'aide d'un moteur (non représenté), et est facilité par la mise en oeuvre d'une pluralité de roues 35.

Un système de verrouillage (non représenté) de la position du dispositif d'amarrage 1 sur le rail 31 est mis en oeuvre afin de sécuriser le dispositif et d'éviter les déplacements latéraux du dispositif d'amarrage 1 dus à la houle, notamment.

Par ailleurs, le dispositif d'amarrage 1 comprend un châssis 4 portant les moyens de verrouillage 5, le châssis 4 étant solidaire du chariot 32.

Le châssis 4 comprend un montant central 41 s'étendant perpendiculairement au chariot 32, et porte des moyens de centrage/guidage qui se présentent sous la forme de deux parois latérales, ou volets, 42. Ces volets 42 sont de forme rectangulaire et s'étendent vers le navire à amarrer en formant un V.

Les volets 42 du châssis 4 sont montés fixes sur le montant 41 par le biais d'au moins un élément de support 43. Ici, chacun des volets 42 est solidaire du montant 41 par l'intermédiaire de deux éléments de support 43 comprenant une base 431 et deux branches 432 (formant un Y). Plus précisément, les volets 42 sont solidaires des branches 432 de l'élément de support 43.

Les volets 42 sont destinés à diriger et/ou centrer les moyens d'engagement 20 portés par le navire 2 vers les moyens de verrouillage 5 disposés sur le ponton 3.

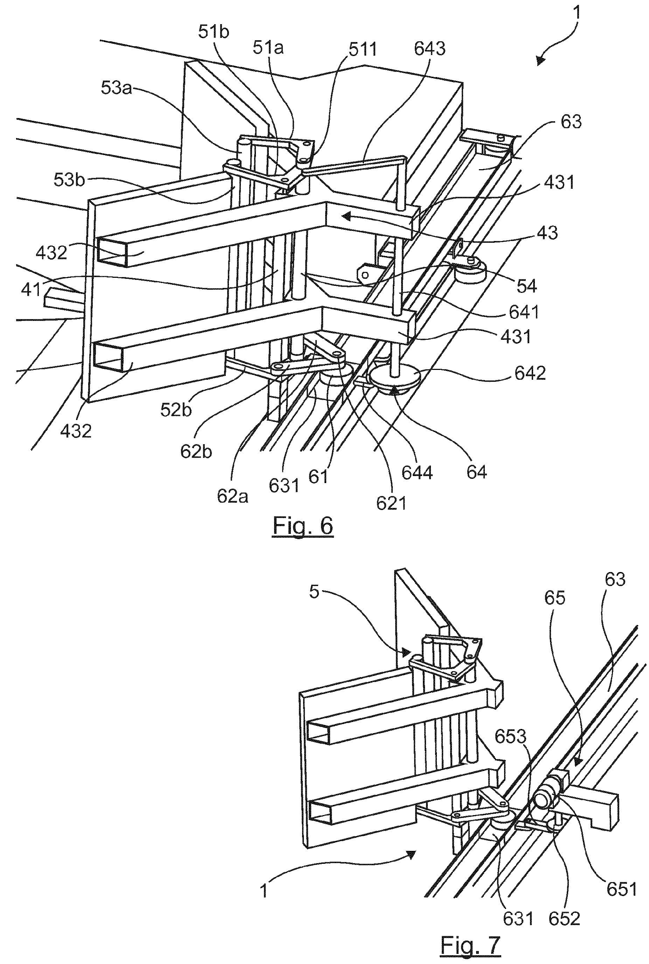

Le châssis 4 présente, à l'arrière du montant 41, et plus précisément à la jointure des deux branches 432 de l'élément de support 43, une ouverture circulaire dans laquelle est monté un tube 54 portant les mâchoires 50a, 50b, comme illustré sur la figure 6.

Dans ce deuxième mode de réalisation, la structure des mâchoires 50a, 50b est identique à celle décrite dans le premier mode de réalisation.

Les moyens de verrouillage 5 mettent ainsi en oeuvre deux mâchoires 50a 50b qui sont solidaires du châssis 4 par le biais de deux paires d'équerres 51a, 51b et 52a, 52b.

Les équerres supérieures 51a, 51b sont mobiles en rotation l'une par rapport à l'autre par le biais d'une liaison pivot, ou axe de rotation, 511 située à une première extrémité de chacune des équerres supérieures 51a, 51b.

Les équerres inférieures 52a, 52b sont, de la même façon, mobiles en rotation l'une par rapport à l'autre par le biais d'une liaison pivot, ou axe de rotation, 521 (non visible), située à une première extrémité de chacune des équerres inférieures 52a, 52b.

La seconde extrémité des équerres inférieures 52a, 52b et des équerres supérieures 51a, 51b sont reliées entres elles par l'intermédiaire d'une barre d'accroche 53a, 53b.

Les équerres et les barres d'accroche forment ainsi les mâchoires 50a et 50b.

Le tube porteur 54, coopérant avec le châssis 4, permet de relier l'axe de rotation 511 des équerres supérieures 51a et 51b à l'axe de rotation 521 des équerres inférieures 52a et 52b. Un tel tube porteur 54 permet en outre de rigidifier/renforcer la structure des moyens de verrouillage 5.

Le dispositif d'amarrage 1 comprend des moyens d'actionnement 6 des moyens de verrouillage 5 comprenant un élément de support (ou galet) 61 sur lequel sont montés articulés autour d'un même axe de rotation 621 deux leviers, ou bielles, 62a et 62b, qui s'étendent, en formant un V d'angle variable, en direction du navire 2 à amarrer.

Les bielles 62a, 62b sont respectivement solidaires à leur autre extrémité d'une équerre inférieure 52a, 52b. Ces équerres 52a, 52b sont montées mobiles en rotation sur les bielles 62a, 62b respectivement autour d'un axe de rotation qui est situé au voisinage de leur coin/angle droit.

L'élément de support 61 est solidaire d'une portion mobile 631 d'un profilé 63 s'étendant parallèlement au bord du ponton 3. La portion mobile 631 est apte à se décaler, c'est-à-dire, à se déplacer en translation perpendiculairement à l'axe longitudinal du profilé 63 selon que l'on souhaite ouvrir ou fermer les mâchoires 50a, 50b.

Dans cet exemple, le profilé 63 est solidaire du chariot 32 et des traverses 34, de manière à permettre le déplacement du dispositif d'amarrage 1 sur le rail 31.

Le déplacement en translation de la portion mobile 631 du profilé 63 est mis en oeuvre par un actionneur 64.

La figure 6 illustre la mise en oeuvre d'un actionneur 64 manuel, ce dernier étant monté pivotant sur chacune des bases 431 de l'élément de support 43 du châssis 4.

Les bases 431 présentent chacune une ouverture circulaire permettant le passage d'un axe 641, s'étendant parallèlement au tube 54. L'axe 641 est solidaire à son extrémité inférieure d'une came 642 et à son extrémité supérieure d'une poignée 643.

Lorsqu'un utilisateur agit sur la poignée 643 de l'actionneur 64, l'axe 641 pivote et entraine en rotation la came 642. La rotation de la came 642 provoque le déplacement de la portion mobile 631 par l'intermédiaire d'une tige 644 reliant la came 642 et la portion mobile 631. Plus précisément, la portion mobile 631, qui est ici disposée selon l'axe longitudinal du profilé 63, se décale et se déplace vers la came 642.

De ce fait, l'angle entre les bielles 62a et 62b diminue ce qui provoque l'ouverture/éloignement des mâchoires 50a, 50b. On comprend que le déplacement de la poignée 643 dans l'autre sens permet de fermer/rapprocher les mâchoires 50a, 50b.

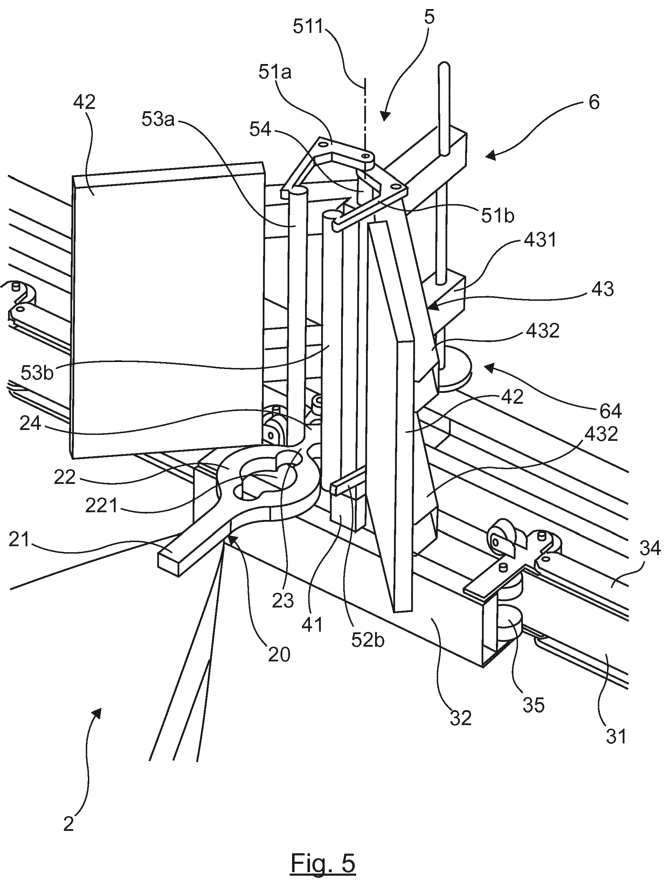

L'actionneur 64 permet ainsi le déplacement des mâchoires 50a, 50b entre la position ouverte, dans laquelle les barres d'accroches 53a, 53b sont éloignées l'une de l'autre autorisant le passage des moyens d'engagement 20 (les équerres supérieures et inférieures formant respectivement un « W ») et une position fermée (comme illustrée sur les figures 5 et 6), dans laquelle les barres d'accroche 53a, 53b sont rapprochées et maintiennent les moyens d'engagement 20 (les équerres supérieures et inférieures formant respectivement un « U »).

L'ensemble comprenant l'actionneur 64, la came d'entrainement 642, la paire de bielles 62a, 62b, l'élément de support 61 et la portion mobile 631, forment les moyens d'actionnement 6 des moyens de verrouillage 5 du dispositif d'amarrage 1.

De la même façon que dans le premier mode de réalisation, les moyens de verrouillage 5 sont destinés à coopérer avec des moyens d'engagement 20.

Comme illustré sur la figure 5, les moyens d'engagement 20 comprennent un axe 21 permettant de relier le navire 2 à amarrer à un anneau 22 qui présente une lumière 221 intérieure. La lumière 221 est destinée à coopérer avec une perche manuelle ou automatique permettant de rapprocher le navire 2 des moyens de verrouillage 5, ou de l'éloigner.

La lumière 221 est formée par une ouverture circulaire prolongée par deux rainures sur les bords opposés situés dans le prolongement de l'axe 21. Bien évidemment, cette lumière 221 peut être circulaire ou présenter une autre forme. Les moyens d'engagement 20 comprennent également une tige, ou portion centrale, 23 de largeur réduite. Une première extrémité de la tige 23 est solidaire de l'anneau 22 et une deuxième extrémité de la tige 23 est reliée à un plot 24.

L'amarrage du navire 2 au ponton 3 est obtenu lorsque le plot 24 est introduit entre les mâchoires 50a, 50b ouvertes et que ces dernières sont rapprochées jusqu'à enserrer (avec un jeu) la tige centrale 23, comme illustré sur la figure 5.

### 6.3 Description d'un troisième mode de réalisation

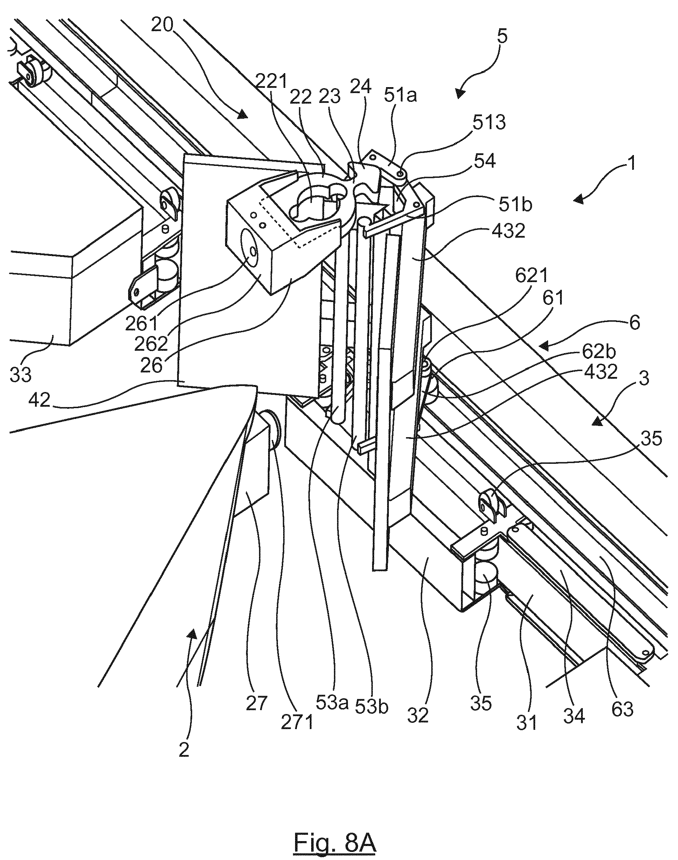

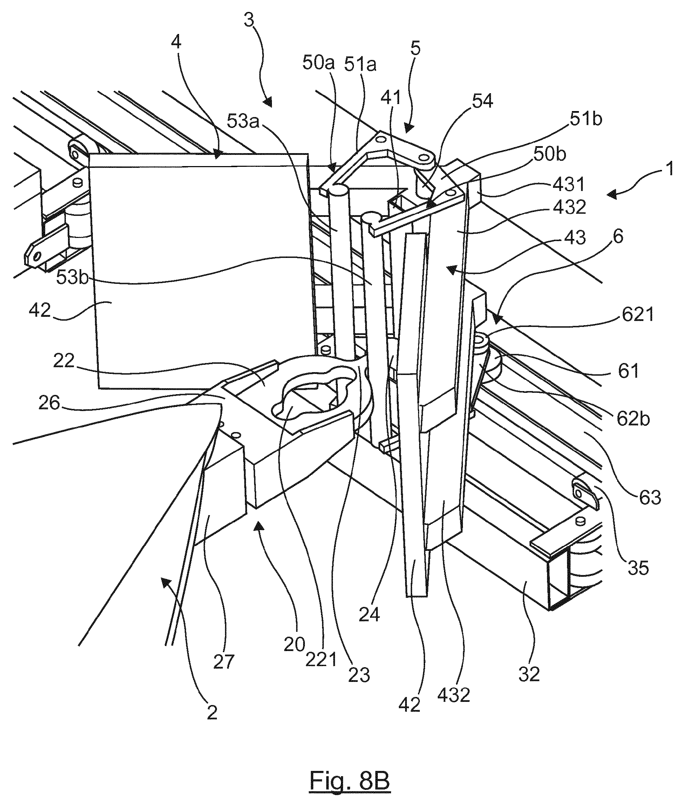

Un dispositif d'amarrage 1 d'un navire 2, selon un troisième mode de réalisation, est représenté sur les figures 8A et 8B.

Ce dispositif d'amarrage 1 comprend des moyens de verrouillage 5 composés de deux mâchoires 50a, 50b solidaires d'un ponton flottant 3, et des moyens d'engagement 20 solidaires du navire 2 à amarrer au ponton 3.

Ce troisième mode de réalisation diffère des deux premiers modes de réalisation décrits précédemment en ce que les mâchoires 50a et 50b des moyens de verrouillage 5 restent en position fermée lors de l'amarrage et du désamarrage du navire 2.

Dans ce troisième mode de réalisation, et de façon similaire au deuxième mode de réalisation, le ponton 3 présente des moyens de déplacement des moyens de verrouillage 5 le long du ponton 3. Ces moyens de déplacement comprennent un rail 31 qui s'étend sur au moins une partie de la longueur du ponton 3. Un chariot coulissant 32 est monté mobile en translation sur le rail 31 et est notamment destiné à supporter les moyens de verrouillage 5 du dispositif d'amarrage 1.

Le chariot 32 est relié en amont et en aval à un appontement flottant ("*catway*" en anglais) 33, s'étendant perpendiculairement au ponton 3, par l'intermédiaire d'une traverse 34 de telle sorte que l'ensemble du dispositif d'amarrage 1 puisse être déplacé le long du ponton 3 sur le rail 31.

Le déplacement de l'ensemble formé par le chariot 32 et les appontements 33 est facilité par la mise en oeuvre d'une pluralité de roues 35.

Un système de verrouillage (non représenté) de la position du dispositif d'amarrage 1 sur le rail 31 est mis en oeuvre afin de sécuriser le dispositif et d'éviter les déplacements latéraux dus à la houle, notamment.

Par ailleurs, le dispositif d'amarrage 1 comprend un châssis 4 solidaire du chariot 32 et destiné à porter les moyens de verrouillage 5.

Le châssis 4 comprend un montant central 41 (visible à la figure 8B) s'étendant perpendiculairement au chariot 32, et présente deux parois latérales, ou volets, 42, de forme sensiblement rectangulaire, s'étendant vers le navire à amarrer et formant un V.

Les volets 42 du châssis 4 sont montés fixes sur le montant 41 par le biais d'au moins un élément de support 43. Ici, chacun des volet 42 est solidaire du montant 41 par l'intermédiaire de deux éléments de support 43 de forme sensiblement en Y (et comprenant une base 431 et deux branches 432). Plus précisément, les volets 42 sont solidaires des branches 432 de l'élément de support 43.

Les volets 42 sont destinés à diriger et/ou centrer les moyens d'engagement 20 portés par le navire 2 vers les moyens de verrouillage 5 disposés sur le ponton 3.

Le châssis 4 présente, à l'arrière du montant 41, et plus précisément à la jointure des deux branches 432 de l'élément de support 43, une ouverture circulaire dans laquelle est monté un tube 54 portant les mâchoires 50a, 50b.

Dans ce troisième mode de réalisation, la structure des mâchoires 50a, 50b est identique à celles décrites dans les deux premiers modes de réalisation.

Les moyens de verrouillage 5 mettent donc en oeuvre deux mâchoires 50a et 50b, solidaires du châssis 4 par le biais de deux paires d'équerres 51a, 51b et 52a, 52b (non visibles).

Les équerres et les barres d'accroche 53a, 53b forment ainsi les mâchoires 50a et 50b.

Comme illustré sur les figures 8A et 8B, les mâchoires 50a, 50b sont en position fermée, les barres d'accroche 53a et 53b étant rapprochées et enserrant les moyens d'engagement 20.

Dans ce troisième mode de réalisation, les moyens d'engagement 20 sont en deux parties démontables. Plus précisément, les moyens d'engagement 20 comprennent un socle, ou première partie fixe, 27 solidaire du navire 2, et une partie amovible, ou deuxième partie, 26 destinée à coopérer avec les mâchoires 50a, 50b des moyens de verrouillage 5.

Le socle 27, qui est fixé au navire 2, présente un pion d'engagement 271 s'étendant dans le prolongement de la pointe avant du navire 2. Le pion d'engagement 271 est destiné à coopérer avec un logement circulaire 261 ménagée dans la base 262 de la partie amovible 26 de façon à solidariser le socle 27 à la partie amovible 26.

La partie amovible 26 présente en outre un anneau 22 comprenant une lumière 221 intérieure. La lumière 221 est destinée à coopérer avec une perche manuelle ou automatique permettant de rapprocher ou d'éloigner le navire 3 des moyens de verrouillage 5.

La lumière 221 est formée par une ouverture circulaire prolongée par deux rainures sur les bords opposés situés dans le prolongement du logement circulaire 261.

Bien évidemment, cette lumière 221 peut être circulaire ou présenter une autre forme.

La partie amovible 26 comprend également une tige, ou portion centrale, 23 de largeur réduite. Une première extrémité de la tige 23 est solidaire de l'anneau 22 et une deuxième extrémité de la tige 23 est reliée à une flèche 24.

L'amarrage du navire 2 au ponton 3 nécessite les opérations suivantes :
- le plaisancier doit d'abord démonter/désolidariser la partie amovible 26 de son socle 27 (comme illustré à la figure 8A) ;
- le plaisancier doit ensuite insérer, par le haut ou par le bas, la flèche 24 des moyens d'engagement 20 entre les mâchoires 50a, 50b fermées des moyens de verrouillage 5. Une fois cette opération effectuée, les mâchoires 50a, 50b sont situées de part et d'autre de la tige 23, et
- le plaisancier doit enfin remonter/solidariser la partie amovible 26 sur le socle 27 du navire 2 (comme illustré à la figure 8B).

Une fois le socle 27 et la partie amovible 26 des moyens d'engagement solidarisés, le navire 2 est solidement amarré au ponton 3.

Le désamarrage du navire 2 nécessite les opérations suivantes :
- le plaisancier doit d'abord démonter/désolidariser la partie amovible 26 de son socle 27 ;
- le plaisancier doit ensuite retirer, par le haut ou par le bas, la flèche 24 des mâchoires 50a, 50b fermées, et
- le plaisancier doit enfin remonter/solidariser la partie amovible 26 sur le socle 27 du navire 2 (comme illustré à la figure 8B).

### 6.4 Description d'un quatrième mode de réalisation

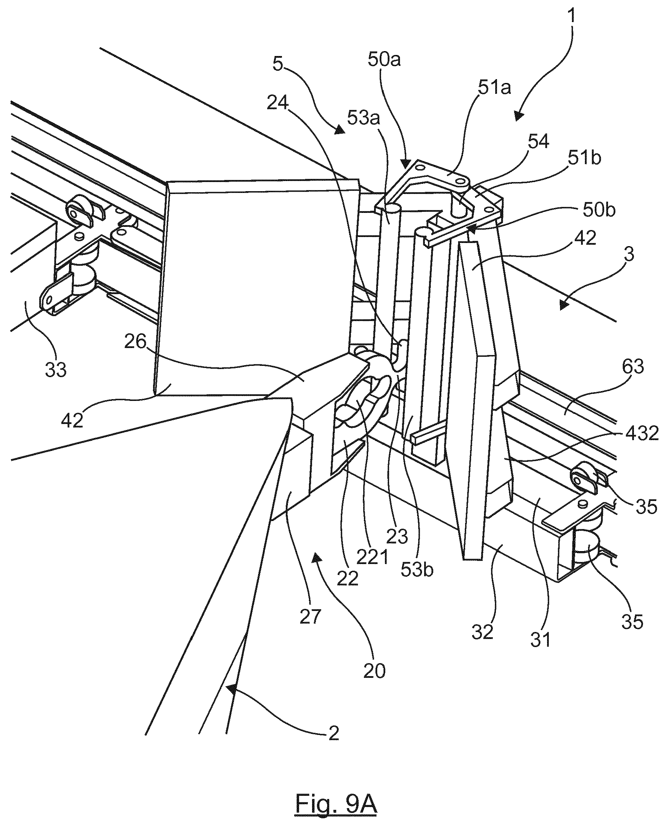

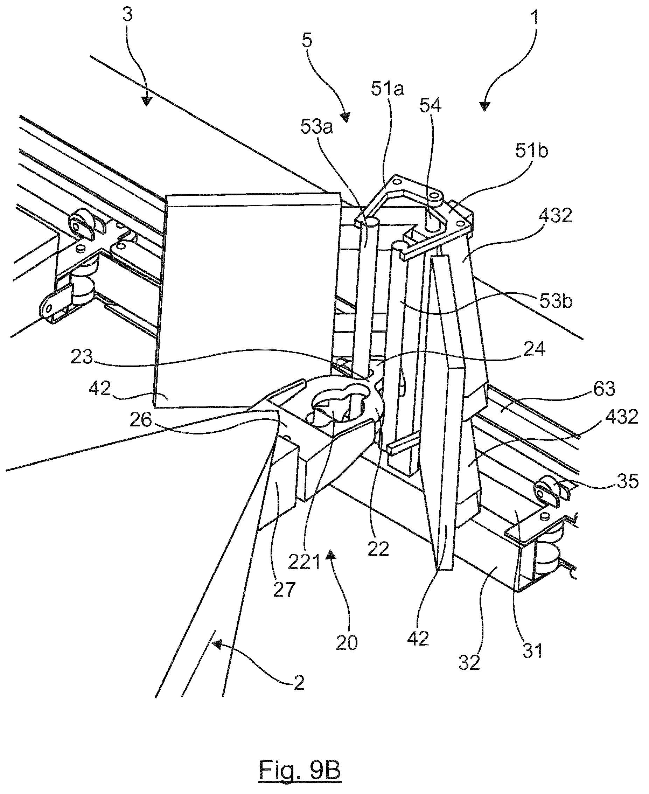

Un dispositif d'amarrage 1 d'un navire 2, selon un quatrième mode de réalisation, est représenté sur les figures 9A et 9B.

Ce dispositif d'amarrage 1 comprend des moyens de verrouillage 5 comprenant deux mâchoires 50a, 50b solidaires d'un ponton flottant 3, et des moyens d'engagement 20 solidaires du navire 2 à amarrer au ponton 3.

.Dans ce quatrième mode de réalisation, les mâchoires 50a et 50b des moyens de verrouillage 5 restent en position fermée lors de l'amarrage et du désamarrage du navire 2.

Ce quatrième mode de réalisation diffère du troisième mode de réalisation décrit précédemment en ce que la deuxième partie 26 des moyens d'engagement 20 du navire 2, destinée à coopérer avec les mâchoires 50a, 50b des moyens de verrouillage 5, est montée mobile en rotation sur le socle, ou partie fixe, 27 qui est solidaire de façon permanente à la pointe avant du navire 2. Cette deuxième partie 26 est montée de façon amovible ou non sur la partie fixe 27.

De façon identique au mode de réalisation précédent, la partie pivotante 26 comprend un anneau 22 présentant une lumière 221 intérieure. La lumière 221 est destinée à coopérer avec une perche manuelle ou automatique permettant de rapprocher ou d'éloigner le navire 2 des moyens de verrouillage 5.

La lumière 221 est formée par une ouverture circulaire prolongée par deux rainures sur les bords opposés situés dans le prolongement du socle 27. Bien évidemment, cette lumière 221 peut être circulaire ou présenter une autre forme.

La partie pivotante 26 comprend également une tige, ou portion centrale, 23 de largeur réduite. Une première extrémité de la tige 23 est solidaire de l'anneau 22 et une deuxième extrémité de la tige 23 est reliée à une flèche 24.

L'amarrage du navire 2 au ponton 3 est simple et nécessite peu d'opérations.

En effet, en position inactive, la partie pivotante 26 est orientée verticalement par rapport à la surface supérieure du pont du navire 2 (comme illustré à la figure 9A). Cette première orientation de la partie pivotante 26 permet à la flèche 24 de se glisser entre les mâchoires 50a, 50b des moyens de verrouillage 5 situés sur le ponton 3.

Lorsque la flèche 24 dépasse les mâchoires 50a et 50b, la partie pivotante 26 pivote de 90° pour passer en position active de manière à bloquer/verrouiller la flèche 24 du navire 2 (comme illustré à la figure 9B). Dans cette position, la tige 23 des moyens d'engagement se situe entre les barres d'accroches 53a, 53b des moyens de verrouillage 5.

Le pivotement de la partie pivotante 26 est obtenu par une action manuelle de l'utilisateur.

Dans une variante, le pivotement peut être automatique ou enclenché par une commande à distance située dans la cabine de pilotage du navire, par exemple.

### 6.5 Autres aspects et variantes

Dans une variante de l'invention, illustrée sur la figure 7, les moyens d'actionnement 6 des moyens de verrouillage 5 mettent en oeuvre un actionneur 65 automatique comprenant un moteur 651. Le moteur 651 est destiné à déplacer la came 652 en rotation, cette dernière agissant sur une tige 653 solidaire de la portion mobile 631 de façon à permettre le décalage de la portion mobile 631 par rapport à l'axe longitudinal du profilé 63.

La fermeture des mâchoires peut être initiée par l'actionneur lorsque des moyens de détection (comprenant un ou plusieurs capteur(s)) déterminent que les moyens d'engagement sont correctement positionnés entre les mâchoires.

Les moyens d'actionnement peuvent être commandés par des moyens de commande à distance situés, par exemple, dans la cabine de commande du navire.

On note que les moyens d'engagement peuvent se situer à l'arrière ou sur les cotés du navire.

Par ailleurs, l'anneau 22 des moyens d'engagement, peut être remplacé par un élément en forme de triangle permettant un guidage optimisé des mâchoires de chaque côté de la tige lors de la fermeture des mâchoires. Dans une variante, les moyens d'engagement 20 peuvent ne pas comprendre d'anneau, le plot 34 étant directement relié à la pointe du navire 2.

Dans une variante des modes de réalisation décrits précédemment, les moyens de verrouillage peuvent être portés par le navire et les moyens d'engagement peuvent être solidaires du ponton.

Le dispositif d'amarrage conforme à l'invention peut notamment être mise en oeuvre en mer, en lac ou en rivière, sur un ponton ou un quai d'un port de plaisance (ou d'un port aménagé le long d'un canal ou d'une rivière navigable), par exemple. Il est particulièrement, mais non exclusivement, adaptée aux bateaux de plaisance.

Il est à noter qu'un même ponton peut être équipé de plusieurs dispositifs d'amarrage, et comprendre par exemple un ou plusieurs dispositifs d'amarrage avec actionneur manuel et/ou un ou plusieurs dispositifs d'amarrage avec actionneur automatique.

## Revendications

1. Dispositif d'amarrage (1) d'un navire (2) à un poste d'amarrage (3) comprenant :
- des moyens d'engagement (20) portés par ledit navire (2) ou ledit poste d'amarrage (3), et
- des moyens de verrouillage (5) portés par ledit poste d'amarrage (3) ou ledit navire (2) respectivement,
lesdits moyens de verrouillage (5) comprenant deux barres d'accroche (53a, 53b) espacées et s'étendant verticalement, au moins une partie des moyens d'engagement (20) étant aptes à venir s'engager entre les deux barres de façon à être maintenus par lesdits moyens de verrouillage (5) et à permettre l'amarrage du navire (2) au poste d'amarrage (3),
**caractérisé en ce que** lesdites barres d'accroche (53a, 53b) sont mobiles l'une par rapport à l'autre entre une position de verrouillage dans laquelle les barres d'accroche (53a, 53b) sont rapprochées et maintiennent lesdits moyens d'engagement (20), et une position de déverrouillage dans laquelle les barres d'accroche (53a, 53b) sont écartées permettant le retrait ou l'insertion desdits moyens d'engagement (20).

2. Dispositif d'amarrage (1) selon la revendication 1, **caractérisé en ce que** lesdits moyens d'engagement (20) comprennent une première portion d'extrémité (24) reliée par une portion centrale (23) de largeur réduite à une deuxième portion d'extrémité (21, 22), cette dernière étant reliée au navire (2) ou au poste d'amarrage (3).

3. Dispositif d'amarrage (1) selon la revendication 2, **caractérisé en ce que** ladite deuxième portion d'extrémité (22) présente une lumière (221) destinée à coopérer avec une perche d'amarrage ou d'éloignement du navire (2).

4. Dispositif d'amarrage (1) selon la revendication 2 ou 3, **caractérisé en ce que** dans la position de verrouillage des barres d'accroche (53a, 53b), ces dernières sont situées de part et d'autre de ladite portion centrale (23).

5. Dispositif d'amarrage (1) selon l'une des revendications 2 à 4, **caractérisé en ce que** ladite première portion d'extrémité (24, 25) est disposée sur un premier côté des barres d'accroche (53a, 53b), l'écart entre les barres d'accroche (53a, 53b) dans la position de verrouillage étant inférieur à la largeur de ladite première portion d'extrémité (24, 25).

6. Dispositif d'amarrage (1) selon l'une des revendications 2 à 5, **caractérisé en ce que** l'écart entre les barres d'accroche (53a, 53b) dans la position de verrouillage est supérieur à la largeur de ladite portion centrale (23).

7. Dispositif d'amarrage (1) selon l'une des revendications 2 à 6, **caractérisé en ce que** l'écart entre les barres d'accroche (53a, 53b) dans la position de verrouillage est inférieur à la largeur de ladite deuxième portion d'extrémité (22).

8. Dispositif d'amarrage (1) selon l'une des revendications 1 à 7, **caractérisé en ce que** chaque barre d'accroche (53a, 53b) porte à au moins une de ses extrémités une équerre (51a, 52a) montée pivotante avec une équerre correspondante (51b, 52b) de l'autre barre d'accroche (53b, 53a), les barres d'accroche (53a, 53b) et les équerres formant ainsi deux mâchoires (50a, 50b) mobiles.

9. Dispositif d'amarrage (1) selon la revendication 8, **caractérisé en ce que** lesdits moyens de verrouillage (5) comprennent des moyens d'actionnement (6) agissant sur au moins une équerre de chaque mâchoire (50a, 50b).

10. Dispositif d'amarrage (1) selon la revendication 9, **caractérisé en ce que** lesdits moyens d'actionnement (6) comprennent une paire de bielles (62a, 62b) montées pivotantes autour d'un même axe (621) à une première de leurs extrémités, chacune desdites bielles (62a, 62b) étant montée pivotante, à une deuxième extrémité, sur une équerre distincte d'une desdites mâchoires.

11. Dispositif d'amarrage (1) selon la revendication 10, **caractérisé en ce que** lesdites bielles (62a, 62b) sont montées pivotantes à leur première extrémité sur des moyens de support (631) mobiles en translation de manière à faire varier l'angle d'ouverture entre les deux bielles (62a, 62b).

12. Dispositif d'amarrage (1) selon la revendication 11, **caractérisé en ce que** les moyens de support (631) sont reliés à une came d'actionnement (642, 652) par l'intermédiaire d'une tige (644, 653).

13. Dispositif d'amarrage (1) selon la revendication 12, **caractérisé en ce que** ladite came (653) est déplacée par un moteur (651).

14. Dispositif d'amarrage (1) selon la revendication 13, **caractérisé en ce que** ladite came (642) est déplacée par une poignée (643) reliée à ladite came (642) par un axe (641).

15. Dispositif d'amarrage (1) selon la revendication 1, **caractérisé en ce que** lesdits moyens de verrouillage (5) sont portés par au moins un chariot (32) apte à se déplacer en translation sur un rail (31), ledit rail étant monté fixe sur le poste d'amarrage (3).

16. Ponton comprenant un ou plusieurs dispositifs d'amarrage selon l'une des revendications 1 à 15.
